# EUROPÄISCHE PATENTANMELDUNG

(11) **EP 1 217 075 A2**
(43) Veröffentlichungstag der Anmeldung: **26.06.2002**
(21) Anmeldenummer: 01100488.4
(22) Anmeldetag: 09.01.2001
(51) Int. Cl.: C12P 5/02, C12M 1/107, C10L 3/06, C05F 1/00

(54) **Verfahren zur Verwertung von Tierkadavern**

(30) Priorität: 20.12.2000 DE 10063912
(71) Anmelder: ReDeLa HERSTELLUNG UND VERTRIEB VON BAUSTOFFEN GmbH, 80543 Pöhl (DE)
(72) Erfinder:
(74) Vertreter: Bockermann, Rolf, Dipl.-Ing.

(57) **Zusammenfassung**

Bei diesem Verfahren werden nach einer Zerkleinerung der Tierkadaver diese zusammen mit einer Flüssigkeit, wie z.B. Wasser, zu einem gärfähigen Produkt vermengt. Daraufhin wird das Produkt unter Druck und entsprechender Temperatur sterilisiert. Das sterilisierte Produkt wird anschließend in einer Biogasanlage vergast. Das bei diesem Prozess entstehende Gas wird einem Kraftwerk zur Energieumwandlung und die ausgegorene Flüssigkeit der Weiterverarbeitung, wie beispielsweise der Landwirtschaft als Dünger, zugeführt.

## Beschreibung

Es zählt zum Stand der Technik, tote Tiere (Tierkadaver), wie z.B. Rinder oder Schweine, zu zerkleinern und diese breiige Masse anschließend mit Wasser zu vermengen. Dieses Gemisch wird dann sterilisiert, anschließend getrocknet und letztlich verbrannt.

Neben der Verbrennung des getrockneten mehlartigen Erzeugnisses ist es auch bekannt, das Erzeugnis als Futtermittel bereit zu stellen.

Aufgrund des Sachverhalts, dass in den Tierkadavern den Menschen schädigende Krankheitserreger enthalten sein können, wird die Bereitstellung des mehlartigen Erzeugnisses als Futtermittel neuerdings untersagt und die Verbrennung des Erzeugnisses vorgeschrieben.

Mit der Trocknung des Erzeugnisses aus Tierkadavern und Wasser und der anschließenden Verbrennung des mehlartigen Erzeugnisses ist jedoch eine erhebliche Belastung der Volkswirtschaft verbunden.

Der Erfindung liegt ― ausgehend vom Stand der Technik ― die Aufgabe zugrunde, ein die Volkswirtschaft nicht belastendes Verfahren zur Verwertung von Tierkadavern vorzuschlagen.

Die Lösung dieser Aufgabe besteht in den Merkmalen des Patentanspruchs 1.

Danach werden die Tierkadaver nach ihrer Zerkleinerung mit einer Flüssigkeit, wie z.B. Wasser, zunächst zu einem gärfähigen Produkt vermengt. Im Anschluss daran wird dieses Produkt unter Druck sterilisiert, so dass etwa vorhandene Krankheitserreger mit Sicherheit abgetötet werden. Das sterilisierte Produkt wird dann einer Biogasanlage zugeführt und hier vergast. Das bei der Vergasung entstehende Gas kann anschließend einem Kraftwerk, insbesondere einem Blockheizkraftwert, zugeführt werden, wo Strom und/oder Wärme und/oder Heißwasser erzeugt wird. Die ausgegorene Flüssigkeit wird der Weiterverwertung zugeleitet. Diese Weiterverwertung kann aufgrund der Sterilisation beispielsweise auch in der Bereitstellung als Dünger für die Landwirtschaft bestehen. Dazu ist es möglich, die Flüssigkeit zu sammeln und entsprechend einer umweltgerechten Landwirtschaft in den dafür vorgesehenen Jahreszeiten und entsprechend dem Düngerbedarf auszubringen.

In einer Weiterbildung des erfindungsgemäßen Verfahrens sehen die Merkmale des Patentanspruchs 2 vor, dass das sterilisierte Produkt zusammen mit anderen, gegebenenfalls ebenfalls sterilisierten, Biomassen in Form von organischen Abfällen, wie Jauche, Gülle, Hühnerkot, Maissilage usw., vergast wird.

Wissenschaftliche Untersuchungen und Erkenntnisse haben gezeigt, dass die Abtötung von Krankheitserregern im Rahmen der Sterilisation einwandfrei gewährleistet wird, wenn gemäß Patentanspruch 3 die Sterilisation während einer Verweildauer von ca. 20 Minuten unter einem Druck von etwa 2,5 bar bei einer Temperatur von ca. 135 °C durchgeführt wird.

## Patentansprüche

1. Verfahren zur Verwertung von Tierkadavern, bei welchem nach einer Zerkleinerung der Tierkadaver diese zusammen mit einer Flüssigkeit, wie z.B. Wasser, zu einem gärfähigen Produkt vermengt werden, worauf das Produkt unter Druck sterilisiert und anschließend in einer Biogasanlage vergast wird, und dass dann das Gas einem Kraftwerk zur Energieumwandlung und die ausgegorene Flüssigkeit der Weiterverarbeitung zugeführt werden.

2. Verfahren nach Patentanspruch 1, bei welchem das sterilisierte Produkt zusammen mit anderen Biomassen vergast wird.

3. Verfahren nach Patentanspruch 1 oder 2, bei welchem die Sterilisation während einer Verweildauer von ca. 20 Minuten unter einem Druck von etwa 2,5 bar bei einer Temperatur von ca. 135 °C durchgeführt wird.
